# EUROPÄISCHE PATENTSCHRIFT

(11) **EP 0 770 685 B1**
(45) Veröffentlichungstag und Bekanntmachung des Hinweises auf die Patenterteilung: **04.04.2001**
(21) Anmeldenummer: 96115271.7
(22) Anmeldetag: 24.09.1996
(51) Int. Cl.: C12P 7/62, C07C 69/003

(54) **Verfahren zur Herstellung von trans-2, cis-4-Decadiensäureethylester**
Process for the preparation of trans-2, cis-4-decadienic acid ethylester
Procédé pour la préparation de l'ester éthylique d'acide trans-2, cis-4-décadiénique

(30) Priorität: 06.10.1995 DE 19537235
(43) Veröffentlichungstag der Anmeldung: 02.05.1997
(73) Patentinhaber: HAARMANN & REIMER GMBH, D-37601 Holzminden (DE)
(72) Erfinder: Gatfield, Ian, Dr., 37671 Höxter (DE); Kindel, Günter, 37671 Höxter (DE)
(74) Vertreter: Petrovicki, Wolfgang, Dr.

(56) Entgegenhaltungen:
- DE-A- 2 439 059
- US-A- 3 953 377
- LIEBIGS ANN. CHEM., Nr. 2, 1982, WEINHEIM/BRD, Seiten 363-365, XP000616044 BESTMANN H.J. UND SÜSS J.: "Eine neue stereoselektive Synthese des "Birnenesters" (2E,4Z)-2,4-Decadiensäure-ethylester"
- J.AGRIC. FOOD CHEM. , Bd. 40, Nr. 10, 1992, Seiten 1925-1929, XP000616014 TAKEOKA G.R. ET AL.: "Volatile Constituents of Asian Pear (Pyrus serotina)"

## Beschreibung

Die Erfindung betrifft ein Verfahren zur Herstellung von trans-2,cis-4-Decadiensäureethylester (nachfolgend "DDSE") aus anderen Estern der trans-2, cis-4-Decadiensäure (nachfolgend "DDS"). Das Verfahren gestattet die Herstellung von natürlichem DDSE.

DDSE ist ein Geschmacksstoff, der zum größten Teil für das Aroma von Williamsbirnen verantwortlich ist. Er wird in naturidentischer Form durch eine aufwendige Synthese gewonnen. Ausgangsprodukt hierfür ist das cis-1-Heptenylbromid, welches in den entsprechenden Lithium-Kupfer-Komplex überführt und schließlich mit Propionsäureethylester zum naturidentischen Ethylester umgesetzt wird, der als grün, fruchtig, saftig, typisch Birne bezeichnet wird. Der auf diese Weise hergestellte DDSE ist allerdings kein Naturstoff in lebensmittelrechtlichem Sinn und darf deshalb auch nicht als natürlicher Aromastoff bezeichnet werden.

Stillingiaöl ist ein aus den Früchten des in China heimischen Talgbaumes (Stillingia sebifera oder Sapium sepiferum, Euphorbiaceae) stammendes Pflanzenfett. Neben etwa 7 Gew.-% Palmitinsäure und etwa 70 Gew.-% C₁₈-Fettsäuren (etwa 40 Gew.-% Linolensäure) enthält es etwa 5 Gew.-% DDS in Form der Glyceride.

Daraus kann durch basenkatalysierte Umesterung mit Ethanol DDSE gewonnen werden (siehe z.B. DE-A-24 39 059); jedoch ist dies in lebenmittelrechtlichem Sinne kein natürliches Verfahren, so daß das so gewonnene Produkt nicht natürlicher Aromastoff genannt werden darf.

Eigene Versuche zur Gewinnung der DDS aus dem Stillingiaöl unter natürlichen Bedingungen schlugen zunächst fehl. So haben wir z.B. versucht, durch die Behandlung mit überhitztem Wasserdampf die natürlichen Triglyceride zu spalten. Hierbei muß eine so hohe Temperatur angewendet werden (> 200°C/ 5 Stunden, ca. 80 bis 90 % Spaltung der Lipide), daß die empfindliche DDS oxidiert wird und/oder polymerisiert (Gehalt nach GC <0,2 % Decadiensäure).

Eigene Versuche mit Esterasen und Lipasen, die DDS durch Hydrolyse der Esterbindung freizusetzen, schlugen entweder völlig fehl oder ergaben sehr kleine Ausbeuten der gewünschten Säure. Die getesteten Esterasen und Lipasen wiesen also offenbar eine zu geringe Spezifität gegenüber DDS auf.

Eigene Versuche zur Bildung des Ethylesters durch eine enzymatische Umesterung mittels gewöhnlicher Lipasen und Esterasen verliefen vermutlich wegen deren fehlender Spezifität ebenfalls erfolglos. Zum Beispiel ist die Esterase 30.000 aus Mucor miehei von Gist-Brocades im allgemeinen in der Lage, Ester gesättigter aliphatischer Carbonsäuren mit guten Ausbeuten umzuestern. Mit Stillingiaöl ergab sich innerhalb von 8 Tagen dagegen ein Rohprodukt mit einem DDSE-Gehalt von weniger als 0,5 Gew.-%. Die Glyceride anderer Fettsäuren, die Bestandteile des Stillingiaöls sind, werden allerdings glatt zu den entsprechenden Ethylestern umgeestert.

Weitere Versuche mit Lipase PS von Pseudomonas spezies (Amano) sowie Lipase B von Candida cylindracea (Biocatalysts) waren ebenfalls erfolglos. Beide Enzyme eignen sich sonst bei Veresterungen sehr gut. Die Umsetzung von Kokosfett mit Ethanol in Anwesenheit von Lipase B führt z.B. ganz eindeutig zur Freisetzung von Ethylcaprylat und Ethylcaprinat in guten Ausbeuten.

Weitere Versuche mit den Lipasen von Pseudomonas fluorescens, Chromobacterium viscosum sowie Pankreaslipase schlugen ebenfalls fehl.

Überraschenderweise wurde gefunden, daß Lipase aus Candida antarctica in der Lage ist, die DDS-glyceride des Stillingiaöls in Gegenwart von Ethanol zu DDSE umzuestern - und zwar in guter Ausbeute.

Die Lipase kann in Form der Mikroorganismen selbst, eines Extrakts derselben oder vorzugsweise als freies oder immobilisiertes Enzym eingesetzt werden. Es kann beispielsweise bei der Firma Novo Nordisk A/S, DK-2880 Bagsvaard, Dänemark, unter der Bezeichnung Novozym® 435 bezogen werden. Dabei handelt es sich um eine Triacylglycerinhydrolase (IUB-Nr. 3.1.1.3, CAS-Nr. 9001-62-1) mit ca. 7000 Propyllaurat-Einheiten pro Gramm (Bestimmung: 60°C/15 Minuten, Substrat: Laurinsäure-1-propylester).

Lipase B aus Candida antarctica wird bevorzugt.

Die Geschwindigkeit der Umsetzung hängt u.a. vom Enzym/Substrat-Verhältnis ab. Wie aus Tabelle 4 der Beispiele zu sehen ist, erhält man nach 2 Tagen mit 20 Gew.-% Enzym, bezogen auf Stillingiaöl, eine fast vollständige Überführung der DDS-glyceride in DDSE. Bei geringeren Enzym/Substrat-Verhältnissen dauert die

Umsetzung entsprechend länger.

Die Umsetzungsgeschwindigkeit ist temperaturabhängig und wird durch stöchiometrische Überschüsse an Ethanol nicht wesentlich beeinflußt.

Die Lipase läßt sich wiederholt für viele Zyklen einsetzen, ohne daß sich die erwünschte Aktivität merklich verändert.

DDSE läßt sich aus dem rohen Reaktionsgemisch gut durch Destillation isolieren. Das erhaltene Produkt besitzt eine Reinheit von über 90 Gew.-% und enthält noch etwa 6 Gew.-% trans-2, cis-4, cis-7-Decatriensäureethylester; das erhaltene Produkt entspricht dem naturidentischen DDSE geschmacklich sehr genau. Der Decatriensäureethylester entsteht durch Umesterung des in Stillingiaöl enthaltenen Decatriensäureglycerids.

Gegenstand der Erfindung ist also ein Verfahren zur Herstellung von DDSE durch enzymatische Umesterung anderer Ester der DDS mit Lipase aus Candida antarctica in Gegenwart von Ethanol.

Unter "anderen Estern" werden vornehmlich die in Stillingiaöl vorkommenden Ester der DDS verstanden, die hauptsächlich Glyceride enthalten.

Bei Umesterung natürlicher DDS-Ester mit natürlichem Ethanol erhält man natürlichen DDSE.

Gegenstand der Erfindung ist weiterhin durch enzymatische Umesterung erhaltener trans-2, cis-4-Decatriensäureethylester gemäß der Patentansprüche 4 und 5.

Die erfindungsgemäße Umesterung findet vorzugsweise in Abwesenheit von Wasser statt. Andere Lösungsmittel können verwendet werden, insbesondere wenn sie sich gegenüber den Enzymen indifferent verhalten, wie z.B. Hexan. Die erfindungsgemäße Umesterung wird vorzugsweise bei Temperaturen zwischen 20 und 60°C durchgeführt.

Die Reaktionszeit hängt von der verwendeten Menge und der Aktivität der Esterase ab und beträgt etwa 24 bis 72 Stunden. Es kann aber vorteilhaft sein, die Reaktion schon früher, z.B. nach 12 bis 18 Stunden, wenn der Umsatz etwa 3 bis 4 % beträgt, abzubrechen und die wiedergewonnenen Ausgangsmaterialien erneut einzusetzen.

Die Lipase kann in reiner Form oder auf einem Träger, auf dem es chemisch oder physikalisch gebunden ist, eingesetzt werden. Die Handhabung des gebundenen Enzymes ist wesentlich einfacher. Die Menge des gebundenen Enzyms beträgt, bezogen auf Stillingiaöl, 10 bis 30 Gew.-%, vorzugsweise etwa 20 Gew.-%.

Nach beendeter Umsetzung kann die Lipase durch geeignete Maßnahmen wie Filtrieren oder Dekantieren abgetrennt und ohne erkennbaren Aktivitätsverlust erneut mehrmals eingesetzt werden.

Die nachfolgenden Beispiele erläutern die Erfindung.

### Beispiele

Die Prozentanteile in den folgenden Tabellen beziehen sich auf das durch Destillation Filtration erhaltene Rohprodukt. Die Angaben wurden über die Flächen unter den Gaschromatogramm-Kurven ermittelt.

### Beispiel 1 (Vergleich)

Eine Mischung aus Stillingiaöl (10 g), natürliches Ethanol (1 g) und Enzym (1 g) wurde bei 20°C 3 Tage geschüttelt. Die Enzyme, die verwendet wurden, waren:
1. Esterase 30.000 aus Mucor miehei (Gist Brocades)
2. Lipase PS aus Pseudomonas spezies (Amano)
3. Lipase B aus Candida cylindracea (Biocatalysts)

**Tabelle 1**

| Ethylester der | % DDSE | | |
|---|---|---|---|
| | Enzym 1 | Enzym 2 | Enzym 3 |
| trans-2, cis-4-Decadiensäure | 0,4 | 0,05 | 0,10 |
| Linolensäure | 65,8 | 57,5 | 65,2 |

Die Werte für den Ethylester der Linolensäure zeigen, daß die verwendeten Enzyme durchaus in der Lage sind, eine Umesterung der Hauptfettsäure des Stillingiaöls zu bewerkstelligen.

### Beispiel 2

Eine Mischung aus Stillingiaöl (10 g), natürliches Ethanol (2 g) und Enzym (1 g) wurde unter Stickstoff bei 45°C im geschlossenen Gefäß geschüttelt. Nach 3 bzw. 7 Tagen wurden Proben gezogen und mittels GC auf den Gehalt an DDSE untersucht.

**Tabelle 2**

| Lipase | | | trans-2, cis-4-Decadiensäureethylester GC-Flächen % nach | |
|---|---|---|---|---|
| | | | 3 Tagen | 7 Tagen |
| 1 | SP 523 | (Novo) | 0,35 | 1,15 |
| 2 | SP 524 | (Novo) | 0,10 | 0,10 |
| 3 | SP 525 | (Novo) | 5,60 | 5,55 |
| 4 | Candida cylindracea | (Biocatalysts) | 0,15 | 0,10 |
| 5 | Mucor miehei | (Biocatalysts) | - | - |
| 6 | Pankreatin | (Biocatalysts) | - | - |
| 7 | Pseudomonas fluorescens | (Biocatalysts) | 0,20 | 0,45 |
| 8 | Chromobacterium viscosum | (Biocatalysts) | 0,05 | 0,05 |
| 9 | Novozym 435 | (Novo) | 6,50 | 8,00 |

Daraus geht eindeutig hervor, daß die Fähigkeit, DDSE aus Stillingiaöl durch Umesterung zu erhalten, bei Lipasen die Ausnahme ist. Gute Ausbeuten werden nur bei SP 525 und Novozym 435 erhalten. Das Novozym 435 ist die immobilisierte Lipase B und SP 525 die freie Lipase B von Candida antarctica.

### Beispiel 3

Eine Mischung aus Stillingiaöl (100g), natürlichem Ethanol (20 g) und Novozym 435 (20 g) wurde in einem 500 ml Erlenmeyerkolben mit Stickstoff überlagert und nach dem Verschließen bei jeweils unterschiedlichen Temperaturen geschüttelt. Die Freisetzung des DDSE wurde quantitativ mittels Gaschromatographie bestimmt (siehe Tabelle 3)

**Tabelle 3**

| Temperatur °C | Gehalt an Ethyldecadienoat in % nach 3 Tagen |
|---|---|
| 20 | 2,2 |
| 30 | 2,7 |
| 40 | 4,3 |
| 50 | 4,4 |
| 60 | 4,5 |

Der Versuch wurde bei 45°C wiederholt. Die Proben sind nach 1, 2 und 3 Tagen gezogen worden. Aus Tabelle 4 geht hervor, daß die optimale Inkubationszeit unter den gegebenen Bedingungen etwa 2 Tage beträgt.

**Tabelle 4**

| Inkubationszeit (Tagen) | Gehalt an Ethyldecadienoat in % |
|---|---|
| 1 | 4,1 |
| 2 | 4,6 |
| 3 | 4,6 |

### Beispiel 4

Eine Mischung aus Stillingiaöl (100 g), natürlichem Ethanol (20 g) und Novozym 435 (10 g bzw. 5 g) wurde in einem 500 ml Erlenmeyerkolben mit Stickstoff überlagert, verschlossen und bei 45°C geschüttelt. Der Gehalt an Ethyldecadienoat wurde mittels GC quantifiziert - siehe Tabelle 5.

**Tabelle 5**

| Enzym (g) | Inkubationszeit (Tage) | Gehalt an Ethyldecadienoat % |
|---|---|---|
| 5 | 1 | 0,70 |
| | 2 | 1,50 |
| 10 | 1 | 1,50 |
| | 2 | 2,50 |
| | 3 | 3,20 |
| | 4 | 3,90 |
| | 7 | 4,40 |
| 20∗ | 1 | 4,10 |
| | 2 | 4,60 |

| | | |
|---|---|---|
| ∗ von Beispiel 3 | | |

### Beispiel 5

Eine Mischung aus Stillingiaöl (100 g), natürlichem Ethanol (20 g bzw. 30 g bzw. 40 g) und Novozym 435 (20 g) wurde in einem 500 ml Erlenmeyerkolben mit Stickstoff überlagert, verschlossen und bei 45°C geschüttelt. Proben wurden gezogen und quantitativ analysiert (siehe Tabelle 6).

**Tabelle 6**

| Ethanol (g) | Gehalt an Ethyldecadienoat % |
|---|---|
| 20 | 4,50 |
| 30 | 4,10 |
| 40 | 3,90 |

Unter Berücksichtigung der verschiedenen Verdünnungsgrade läßt sich errechnen, daß die Effektivität der Bildung des Ethylesters in diesem Bereich unabhängig von der Ethanolkonzentration ist. Rechnerisch liegt das äquimolare Verhältnis bei ca. 16 g Ethanol per 100 g Stillingiaöl.

### Beispiel 6

Eine Mischung aus Stillingiaöl (100 g), natürlichem Ethanol (20 g) und Novozym 435 (20 g) wurde in einem 500 ml Erlenmeyerkolben mit Stickstoff überlagert, verschlossen und bei 45°C geschüttelt. Nach 2 bzw. 3 Tagen wurde der Inhalt abfiltriert und das immobilisierte Enzym mit weiterem Stillingiaöl (100 g) und natürlichem Ethanol (20 g) versetzt mit Stickstoff überlagert, verschlossen und geschüttelt. Insgesamt wurden 12 Zyklen mit dem gleichen Enzym gefahren, ohne daß die enzymatische Aktivität merklich nachließ (siehe Tabelle 7).

**Tabelle 7**

| Zyklus | Inkubationszeit (Tage) | Gehalt an Ethyldecadienoat (%) |
|---|---|---|
| 1 | 1 | 4,5 |
| 2 | 1 | 4,2 |
| 3 | 1 | 4,2 |
| 4 | 2 | 4,6 |
| 5 | 2 | 4,2 |
| 6 | 2 | 4,3 |
| 7 | 2 | 4,5 |
| 8 | 3 | 4,4 |
| 9 | 3 | 4,0 |
| 10 | 1 | 4,1 |
| 11 | 1 | 4,2 |
| 12 | 3 | 4,8 |

### Beispiel 7

Vom gesammelten Filtrat aus Beispiel 6 wurden etwa 500 g mit einem Gehalt von 4,3 Gew.-% an Ethyldecadienoat einer destillativen Reinigung unterworfen. Die Destillation wurde so geführt, daß zunächst eine Vorfraktion von etwa 10 Gew.-% der Einsatzmenge isoliert wurde. Diese Vorfraktion wies einen Gehalt an Ethyldecadienoat von etwa 40 Gew.-% auf. Die Wiedergewinnung des wertbestimmenden Inhaltsstoffes betrug an dieser Stelle etwa 95 %. Diese Vorfraktion wurde dann einer Feindestillation unterworfen. Es wurden Fraktionen erhalten, die nach GC eine typische Reinheit an DDSE von 93 Gew.-% und höher aufwiesen. Das so erhaltene Produkt schmeckte sehr sauber und typisch nach Williamsbirne und war geschmacklich dem naturidentischen DDSE ebenbürtig. Die Hauptvenunreinigung war mit etwa 6 % der Ethylester der trans-2, cis-4, cis-7-Decatriensäure, die ebenfalls in Stillingiaöl vorkommt und mit dem verwendeten Enzym umgeestert wird.

### Beispiel 8

Eine Mischung aus Stillingiaöl (5 kg), natürlichem Ethanol (1,0 kg) und Novozym 435 (1,0 kg) wurde in ein 20 l-Glasgefäß mit Stickstoff überlagert und bei 45°C langsam gerührt. Das Gefäß war mit einem Wasserkühler versehen, um Verluste durch Verdampfen zu minimieren. Nach einer Inkubationszeit von jeweils 2 bis 3 Tagen wurde der Ansatz abgebrochen. Das abfiltrierte Enzym wurde mit frischem Stillingiaöl und natürlichem Ethanol versetzt und weiter gerührt. Insgesamt wurden 10 Zyklen durchgeführt, wobei der Gehalt an DDSE von den einzelnen Filtraten zwischen 4,0 und 4,7 Gew.-% variierte.

## Patentansprüche

1. Verfahren zur Herstellung von trans-2, cis-4 -Decadiensäure-ethylester durch enzymatische Umesterung anderer Ester der trans-2, cis-4-Decadiensäure mit Lipase aus Candida antarctica in Gegenwart von Ethanol.

2. Verfahren nach Anspruch 1, wonach als Quelle der anderen Ester Stillingiaöl eingesetzt wird.

3. Verfahren nach Anspruch 1, wonach man in Gegenwart von natürlichem Ethanol umestert.

4. Durch enzymatische Umesterung erhaltendes Produkt, enthaltend trans-2, cis-4-Decadiensäure-ethylester.

5. Natürlicher trans-2, cis-4-Decadiensäure-ethylester, dadurch gekennzeichnet, daß er durch enzymatische Umesterung anderer Ester der trans-2, cis-4-Decadiensäuren mit Lipase aus Candida antarctica in Gegenwart von Ethanol hergestellt wird.

## Claims

1. A process for producing trans-2, cis-4 decadienoic acid ethyl ester by the enzymatic transesterification of other esters of trans-2, cis-4 decadienoic acid with lipase from *Candida antarctica* in the presence of ethanol.

2. A process according to claim 1, in which stillingia oil is used as the source of other esters.

3. A process according to claim 1, in which transesterification is conducted in the presence of natural ethanol.

4. A product which contains trans-2, cis-4 decadienoic acid ethyl ester and which is obtained by enzymatic transesterification.

5. Natural trans-2, cis-4 decadienoic acid ethyl ester, characterised in that it is produced by the enzymatic transesterification of other esters of trans-2, cis-4 decadienoic acid with lipase from *Candida antarctica* in the presence of ethanol.

## Revendications

1. Procédé pour la préparation du trans-2, cis-4-décadiénoate d'éthyle par transestérification enzymatique d'autres esters de l'acide trans-2, cis-4-décadiénoïque à l'aide de la lipase de Candida antarctica en présence d'éthanol.

2. Procédé selon revendication 1, caractérisé en ce que l'on utilise en tant que source des autres esters l'huile de stillingia.

3. Procédé selon revendication 1, selon lequel on transestérifie en présence d'éthanol naturel.

4. Produit obtenu par transestérification enzymatique, contenant du trans-2, cis-4-décadiénoate d'éthyle.

5. Trans-2, cis-4-décadiénoate d'éthyle naturel, caractérisé en ce qu'il est préparé par transestérification enzymatique d'autres esters de l'acide trans-2, cis-4-décadiénoïque à l'aide de la lipase de Candida antarctica en présence d'éthanol.
